# EUROPEAN PATENT SPECIFICATION

(11) **EP 3 455 272 B1**
(45) Date of publication and mention of the grant of the patent: **27.05.2020**
(21) Application number: 17725405.9
(22) Date of filing: 10.05.2017
(51) Int. Cl.: C08G 18/48, C08G 18/66, C08G 18/76, C08G 18/24, C09D 175/04

(54) **THERMOSET POLYISOBUTYLENE-POLYURETHANES AND METHODS FOR MAKING**
WÄRMEHÄRTENDE POLYISOBUTYLEN-POLYURETHANE UND VERFAHREN ZUR HERSTELLUNG
POLYISOBUTYLÈNE-POLYURÉTHANES THERMODURCIS ET LEURS PROCÉDÉS DE FABRICATION

(30) Priority: 10.05.2016 US 201662333958 P
(43) Date of publication of application: 20.03.2019
(73) Proprietor: Cardiac Pacemakers, Inc., St. Paul, Minnesota 55112 (US)
(72) Inventor: DELANEY, JR., Joseph T., Minneapolis Minnesota 55418 (US); KANE, Michael J., St. Paul Minnesota 55113 (US); CUNNINGHAM, Laura, Drogheda Co. Louth (IE)
(74) Representative: Pfenning, Meinig & Partner mbB
(86) International application number: PCT/US2017/031856
(87) International publication number: WO 2017/196913

(56) References cited:
- WO-A1-2010/081132
- WO-A1-2016/007367
- US-A1- 2016 024 340

## Description

### TECHNICAL FIELD

The present invention relates to polymeric materials. More specifically, the invention relates to thermoset polyisobutylene-based polyurethanes, methods for making thermoset polyisobutylene-based, and medical devices containing thermoset polyisobutylene-based polyurethanes.

### BACKGROUND

Polymeric materials are widely used in the field of medical devices. For example, polymeric materials such as silicone rubber, polyurethane, and fluoropolymers are used as coating and/or insulating materials for medical leads, stents, catheters, and other devices.

### SUMMARY

claim 1 is a thermoset polyisobutylene network polymer including a polyisobutylene diol residue, a diisocyanate residue, and at least one crosslinking compound residue selected from the group consisting of a residue of a sorbitan ester and a residue of a branched polypropylene oxide polyol.

In claim 2, the thermoset polymer of claim 1, wherein the polyisobutylene diol residue is present in the polymer in amount of 45% to 97.5% by weight of the polymer, the diisocyanate residue is present in the polymer in an amount of about 2% to about 30% by weight of the polymer, and the at least one crosslinking compound residue is present in the polymer in an amount of about 0.5% to about 25% by weight of the polymer.

In claim 3, the thermoset polymer of either of claims 1 or 2, wherein the diisocyanate residue includes 4,4'-methylene diphenyl diisocyanate residue and 2,4'-methylene diphenyl diisocyanate residue.

In claim 4, the thermoset polymer of claim 3, wherein the 2,4'-methylene diphenyl diisocyanate residue ranges from 1 wt. % to 50 wt. % of the diisocyanate residue.

In claim 5, the thermoset polymer of any of claims 1-4, wherein the crosslinking compound residue is a residue of a sorbitan ester with a hydroxyl functionality greater than 2.

In claim 6, the thermoset polymer of any of claims 1-4, wherein the crosslinking compound residue is a residue of a branched polypropylene oxide polyol with a hydroxyl functionality greater than 2.

In claim 7, the thermoset polymer of any of claims 1-5, and further including a residue of at least one of 1,1,1-tris(hydroxymethyl)propane and 1,2,3-trihydroxypropane.

In claim 8, the thermoset polymer of any of claims 1-6, wherein the polyisobutylene diol residue is a residue of α,ω-bishydroxy-terminated polyisobutylene.

claim 9 is a medical device comprising the thermoset polymer of any of claims 1-8.

In claim 10, the medical device of claim 9, wherein the thermoset polymer forms an electrically insulating and environmentally isolating portion of an electrical feedthrough.

claim 11 is a method of making a medical device including a thermoset polyisobutylene-polyurethane polymer, the method comprising mixing at a temperature ranging from 18°C to 30°C a polyisobutylene diol, a diisocyanate, a polymerization catalyst, and a crosslinking compound to form a liquid mixture, wherein the crosslinking compound including at least one member selected from the group consisting of sorbitan ester having a hydroxyl functionality greater than 2 and a branched polypropylene oxide polyol having a hydroxyl functionality greater than 2; applying the liquid mixture to the medical device, and heating the medical device to cure the liquid mixture and form a thermoset polyisobutylene-polyurethane polymer on the medical device.

In claim 12, the method of claim 11, wherein applying the liquid mixture includes filling a space between a portion of the medical device and at least one electrical conductor with the liquid mixture, and the solid thermoset polyisobutylene-polyurethane polymer electrically insulates the electrical connection from the portion of the medical device.

In claim 13, the method of either of claims 11 or 12, wherein the diisocyanate includes 4,4'-methylene diphenyl diisocyanate ranging between 50 wt. % to 99 wt. % of the diisocyanate, and 2,4'-methylene diphenyl diisocyanate ranging between 1 wt. % and 50 wt. % of the diisocyanate.

In claim 14, the method of any of claims 11-13, further including desiccating the one or more crosslinking compounds before mixing together with the polyisobutylene diol, the diisocyanate, and the polymerization catalyst.

In claim 15, the method of claim 14, wherein the one or more crosslinking compounds further include of at least one of 1,1,1-tris(hydroxymethyl)propane and 1,2,3-trihydroxypropane; and further including mixing together the crosslinking compounds before desiccating the crosslinking compounds.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 depicts an example of a thermoset PIB-PUR in accordance with described embodiments.
FIG. 2 shows comparative lap shear strengths as a function of cure time and cure temperature for a thermoset PIB-PUR using a single crosslinking compound in accordance with described embodiments.
FIG. 3 shows comparative lap shear strength as a function of cure time for thermoset PIB-PUR using a combination of crosslinking compounds in accordance with described embodiments, and in comparison to the thermoset PIB-PUR shown in FIG. 2 for the same cure temperature.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

A more complete understanding of the present invention is available by reference to the following detailed description of numerous aspects and embodiments of the invention. The detailed description of the invention which follows is intended to illustrate but not limit the invention.

In accordance with various aspects of the disclosure, thermoset polyisobutylene polyurethane (also referred to herein collectively as "thermoset polyisobutylene network" or "thermoset PIB-PUR") including at least one crosslinking compound and methods for making the same are disclosed. The at least one crosslinking compound can include at least one of a sorbitan ester and a branched polypropylene oxide polyol.

A thermoset, or thermosetting, polymer is a polymer that cures irreversibly. That is, once cured, a thermoset polymer cannot be reheated and remolded. In contrast, a thermoplastic polymer is a polymer that cures reversibly. That is, it may be heated to soften, remolded, and then hardened by cooling and this process may be repeated indefinitely. Thermoset polymers are able to retain their strength without softening. This is a useful feature, for example, in applications requiring a reliable seal over a range of temperatures.

Thermoset PIB-PUR can include telechelic polyisobutylene, polyol cross-linking compounds to enable network formation, and diisocyanates to link the polyisobutylene to the cross-linking compounds. Medical devices that can be implantable or insertable into the body of a patient and that comprise at least one thermoset PIB-PUR are also disclosed.

Polyurethanes are a family of polymers that are synthesized by reacting polyfunctional isocyanates (e.g., diisocyanates, including both aliphatic and aromatic diisocyanates) with polyols (e.g., macroglycols). Commonly employed macroglycols include polyester diols, polyether diols, polycarbonate diols, polysiloxane diols and α,ω-bishydroxy-terminated polyisobutylene.

In some embodiments, the thermoset PIB-PUR includes one or more polyisobutylene diol residues, one or more diisocyanate residues, and one or more crosslinking compound residues. The relative weight percentages of polyisobutylene residues, diisocyanate residues, and crosslinking compound residues in the thermoset PIB-PUR of the various embodiments can be varied to achieve a wide range of physical and mechanical properties, including Shore Hardness, and to achieve an array of desirable functional performance. In some embodiments, the polyisobutylene residues can be at least as great as 45 wt. %, 50 wt. %, 55 wt. %, or 60 wt. %, or may be no greater than 70 wt. %, 80 wt. %, 90 wt. %, or 97.5 wt. %., or may be present within any range defined between any pair of the foregoing values. For example, in some embodiments, the polyisobutylene residues may be in an amount from 45 wt. % to 97.5 wt. %, from 50 wt. % to 90 wt. %, from 55% to 80%, or from 60 wt. % to 70 wt. %. In some embodiments, the diisocyanate residues can be at least as great as 2 wt. %, 6 wt. %, 10 wt. %, 14 wt. %, or 18 wt. %, or may be no greater than 22 wt. %, 24 wt. %, 26 wt. %, 28 wt. %, or 30 wt. %., or may be present within any range defined between any pair of the foregoing values. For example, in some embodiments, the diisocyanate residues may be in an amount from 2 wt. % to 30 wt. %, from 6 wt. % to 28 wt. %, from 10 wt. % to 26 wt. %, from 14 wt. % to 24 wt. %, or from 18% to 22 wt. %. In some embodiments, the crosslinking compound residues can be at least as great as 0.5 wt. %, 3 wt. %, 6 wt. %, or 10 wt. %, or may be no greater than 18 wt. %, 21 wt. %, 23 wt. %, or 25 wt. %., or may be present within any range defined between any pair of the foregoing values. For example, in some embodiments, the crosslinking compound residues may be in an amount from 0.5 wt. % to 25 wt. %, from 3 wt. % to 23 wt. %, from 6 wt. % to 21 wt. %, or from 10% to 18%. All weight percentages above are by weight of the polymer.

The polyisobutylene residues can vary widely in molecular weight, but can be composed of between 2 and 100 repeat units (monomer units), among other values, and can be incorporated into the thermoset PIB-PUR of the various embodiments in the form of telechelic diol starting materials, such as α,ω-bishydroxy-terminated polyisobutylene.

Diisocyanates for use in forming the thermoset PIB-PUR of the various embodiments include aromatic and non-aromatic (e.g., aliphatic) diisocyanates, and suitable combinations of aromatic and/or non-aromatic diisocyanates. Aromatic diisocyanates may be selected from suitable members of the following, among others: 2,2-, 2-4-, and/or 4,4-methylene diphenyl diisocyanate (MDI), triphenyl methane triisocyanate, 2,4- and/or 2,6-toluene diisocyanate (TDI), 1,5-naphthalene diisocyanate (NDI), para-phenylene diisocyanate, 3,3'-tolidene-4,4'-diisocyanate and 3,3'-dimethyl-diphenylmethane-4,4'-diisocyanate. Non-aromatic diisocyanates may be selected from suitable members of the following, among others: 1,6-hexamethylene diisocyanate (HDI), 4,4'-dicyclohexylmethane diisocyanate (H12MDI), 3-isocyanatomethyl-3,5,5-trimethylcyclohexyl isocyanate (isophorone diisocyanate or IPDI), cyclohexyl diisocyanate, and 2,2,4-trimethyl-1,6-hexamethylene diisocyanate (TMDI).

In some embodiments, a combination of diisocyanate isomers can be used. For example, a combination of 4,4'-methylene diphenyl diisocyanate and 2,4'-methlene diphenyl diisocyanate can be used. The weight ratio of 4,4'-methylene diphenyl diisocyanate to 2,4'-methlene diphenyl diisocyanate in the diisocyanate of the various embodiments can be varied to achieve a diisocyanate ranging in form from solid to liquid at room temperature, resulting in a range of physical and mechanical properties in the resulting thermoset PIB-PUR. For example, the weight ratio of 4,4'-methylene diphenyl diisocyanate to 2,4'-methlene diphenyl diisocyanate in the diisocyanate can be varied from 99:1 to 95:5 to 90:10 to 75:25 to 50:50 to 25:75 to 10:90 to 5:95 to 1:99, more particularly from 95:5 to 90:10 to 80:20 to 70:30 to 65:35 to 60:40 to 50:50, and even more particularly, from about 80:20 to about 50:50. In some embodiments, the diisocyanate can include 4,4'-methylene diphenyl diisocyanate in an amount of 50 wt. % to 99 wt. % of the diisocyanate and 2,4'-methlene diphenyl diisocyanate an amount of 1 wt. % to 50 wt. % of the diisocyanate. In some embodiments, the 4,4'-methylene diphenyl diisocyanate can be 50 wt. % of the diisocyanate and 2,4'-methylene diphenyl diisocyanate can be 50 wt. % of the diisocyanate and the resulting diisocyanate is liquid at room temperature.

Crosslinking compounds are employed to crosslink polyisobutylene-polyurethane polymer chains to provide the thermosetting attribute of the thermoset PIB-PUR. Crosslinking compounds for use in forming the thermoset PIB-PUR of the various embodiments include a sorbitan esters and/or a branched polypropylene oxide polyols. Sorbitan esters may be selected from suitable members of the following, among others: sorbitan monolaurate, polysorbate monolaurate, sorbitan monooleate, and sorbitan sequioleate. Branched polypropylene oxide polyols may be selected from suitable members of the following, among others: Pluracol® PEP450 or Pluracol® 550 available from BASF Corporation of Florham Park, NJ; or Jeffol® FX31-167, Jeffol® FX31-240, or Jeffol® G30-650 available from Huntsman Corporation of The Woodlands, TX. Suitable crosslinking compounds have a hydroxyl functionality greater than 2.

A medical device including a thermoset PIB-PUR in accordance with embodiments of this disclosure can be made by mixing a polyisobutylene diol, a diisocyanate, and one or more crosslinking compounds described above, and a suitable polymerization catalyst together at room temperature, forming a liquid mixture. Room temperature can range from 18°C to 30°C. The liquid mixture can be applied at room temperature to the medical device, and then the medical device heated until the liquid mixture cures, forming the solid, thermoset PIB-PUR. In some embodiments, a cure temperature can be at least as great as 100°C, 110°C, or 120°C, or may be no greater than 130°C, 140°C, or 150°C, or may be present within any range defined between any pair of the foregoing values. For example, in some embodiments, the cure temperature may range from 100°C to 150°C, from 110°C to 140°C, or from 120°C to 130°C. In some embodiments, a cure time can be at least as great as 1 hour, 2 hours, 4 hours, 6 hours, or 8 hours, or may be no greater than 8 hours, 10 hours, 12 hours, 14 hours, or 16 hours, or may be present within any range defined between any pair of the foregoing values. For example, in some embodiments, the cure time may range from 1 hour to 16 hours, from 2 hours to 14 hours, from 4 hours to 12 hours, or from 6 hours to 10 hours.

In some embodiments, the liquid mixture may be applied by filling a space between a portion of the medical device and a least one electrical conductor with the liquid mixture. Once cured, the thermoset PIB-PUR electrically insulates the at least one electrical conductor from the portion of the medical device. In some embodiments, such a structure can form a durable, biocompatible electrical feedthrough that also isolates environments on opposite sides of the feedthrough by substantially resisting chemical and material diffusion through the feedthrough. In some embodiments, the feedthrough substantially resists gas permeation. Such a structure can provide a more versatile and cost effective replacement for traditional ceramic electrical feedthroughs, which require high-purity ceramics, titanium gold brazing, and precious metal conductors to form a biocompatible seal. The thermoset PIB-PUR according to embodiments of this disclosure may form a biocompatible seal as the diisocyanate covalently bonds with implantable materials having stable, non-bioreactive oxides (e.g. titanium, glass).

In other embodiments, the liquid mixture may be applied by injecting into a space in a mold configured to produce a medical device casing. Once cured, the thermoset PIB-PUR may form a flexible, biocompatible device casing that substantially resists chemical and material diffusion to isolate an internal environment defined by the casing from an external environment. In some embodiments, the device casing substantially resists gas permeation. In some applications, such a flexible device casing may provide more patient comfort compared with casings made of more traditional materials, such as titanium.

It is generally desirable that the thermoset PIB-PUR have a generally uniform composition. A continuous, uninterrupted phase of polyisobutylene provides better performance as a diffusion barrier to moisture, oxygen and other contaminants. Achieving this generally good homogeneity requires miscibility of the diisocyanate and the crosslinking compounds with the polyisobutylene diol when forming the liquid mixture. This is challenging because the polyisobutylene diol is so hydrophobic that it is generally not miscible with compounds having multiple functional hydroxyl groups. Hydroxyl groups are polar and may participate in hydrogen bonding, making them hydrophilic and less miscible with the polyisobutylene diol. The crosslinking compounds including the sorbitan esters and the branched polypropylene oxide polyols described above include moieties that are sufficiently hydrophobic to counteract the hydrophilicity of the multiple functional hydroxyl groups. The sorbitan esters include a hydrophobic fatty acid ester moiety. The branched polypropylene oxide polyols include lipophilic branched polypropylene glycol moieties. Thus, the sorbitan esters and branched polypropylene oxide polyols described above are suitable for use in forming a thermoset PIB-PUR in accordance with embodiments of this disclosure. In some embodiments, the crosslinking compounds may be desiccated before being mixed together with the other components to remove as much moisture as possible from the crosslinking compounds, further improving their miscibility with the polyisobutylene diol. Good miscibility of the diisocyanate and the crosslinking compounds with the polyisobutylene diol drives the polymerization reaction more to completion because the OH groups and the NCO groups are more evenly distributed. Uneven distribution may lead to veins of unreacted compounds which may compromise the mechanical strength of the polymer.

In some embodiments, the components of the liquid mixture are miscible at room temperature. The time available to dispense the liquid mixture before it cures enough that it can no longer be processed due to increased viscosity or solidification, decreases significantly if the liquid mixture must be heated above room temperature to achieve miscibility between the components. Employing a combination of diisocyanate isomers that are liquid at room temperature, as described above, permits formation and use of the liquid mixture at room temperature.

The polyisobutylene diol may be a telechelic polyisobutylene diol formed from by carbocationic polymerization beginning with a difunctional initiator compound, such as 5-*tert*-butyl-1,3-*bis*(1-methoxy-1-methylethyl)benzene (hindered dicumyl ether). The resulting compound may be a polyisobutylene diol according to Formula I: 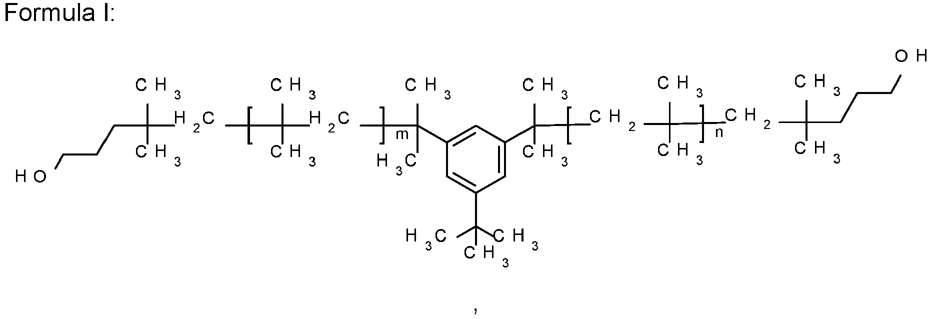 where *m* and *n* are the number of repeating isobutylene monomer segments, and the sum of *m* and *n* ranges from 2 to 100.

The crosslinking compound includes more than two functional hydroxyl groups, as shown in the example of polysorbate monolaurate, which has three functional hydroxyl groups according to Formula II: 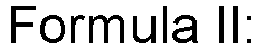 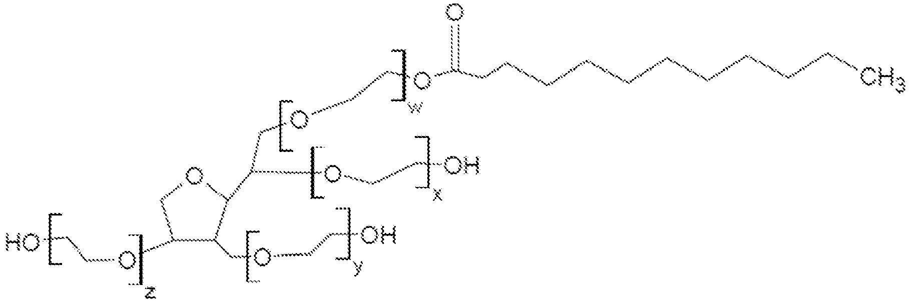 where *w, x, y,* and *z* are the number of repeating propylene oxide monomer segments, and the sum of *w, x, y,* and *z* is 20.

As describe above, the diisocyanate can be a combination of diisocyanate isomers 4,4'-methylene diphenyl diisocyanate according to Formula III: 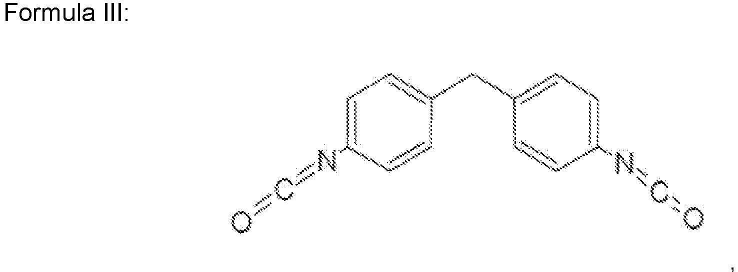 and 2,4'-methlene diphenyl diisocyanate according to Formula IV:

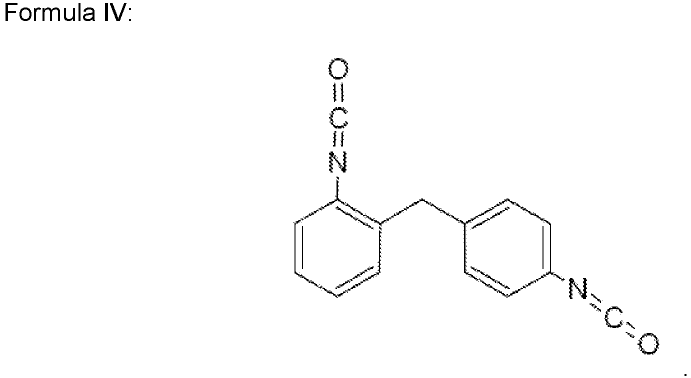

Each of the isocyanate groups may react with a hydroxyl group of the polyisobutylene diol, or with any functional hydroxyl groups of the crosslinking compound, such as the three functional hydroxyl groups available on the polysorbate monolaurate. In this way, the polyisobutylene-polyurethane chains are linked together, forming the thermoset PIB-PUR.

The polymerization catalyst increases the rate of urethane linkage formation and significantly reduces the time for substantial completion of the polymerization reaction. A suitable catalyst must be phase compatible with both the hydrophilic polyisobutylene diol and with the other, less hydrophobic components used in forming the thermoset PIB-PUR. A suitable catalyst must also promote comparable reaction rates between the diisocyanate and each of the polyisobutylene diol and the crosslinking compounds in order to form a thermoset PIB-PUR having residues from each substantially uniformly distributed along the polymer. Suitable polymerization catalysts include tin(II) 2-ethylhexanoate (stannous octoate) and 2,6-dimethylpyridine as described in U.S. provisional patent application number 62/268732, "Polyisobutylene-Polyurethanes and Medical Devices Containing the Same," filed December 17, 2015.

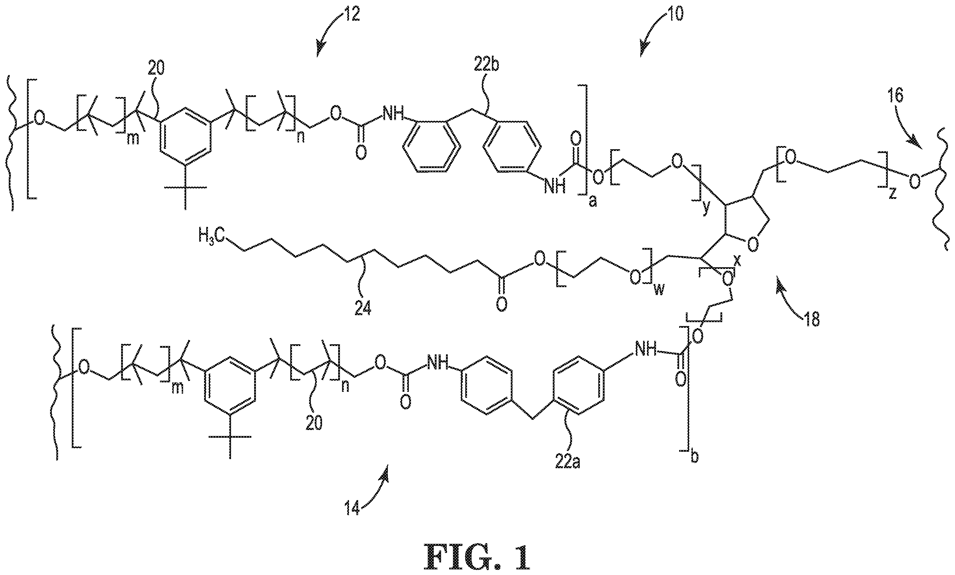

FIG. 1 depicts a portion of an exemplary thermoset PIB-PUR polymer 10 including three polyisobutylene-polyurethane chains 12, 14, 16 linked together by a crosslinking compound residue, polysorbate monolaurate residue 18. For ease of illustration, only two of the polyisobutylene-polyurethane chains 12, 14 are shown. The polyisobutylene-polyurethane chains 12, 14, 16 each include a polyisobutylene diol residue 20 and a diisocyanate residue including either (shown) or both of 4,4'-methylenediphenyl diisocyanate residue 22a and 2,4'-methlene diphenyl diisocyanate residue 22b. As depicted in FIG. 1, the functional hydroxyl groups of the polysorbate monolaurate 18 form urethane linkages with the diisocyanates 22a, 22b. The polysorbate monolaurate residue 18 links together three polyisobutylene-polyurethane chains 12, 14, 16, forming the thermoset PIB-PUR 10.

As also shown in FIG. 1, the polysorbate monolaurate residue 18 includes a fatty acid ester moiety 24. The fatty acid ester moiety 24 provides a high degree of hydrophobicity to the polysorbate monolaurate, countering the polar nature of the three functional hydroxyl groups. Without the fatty acid ester moiety 24, the polysorbate monolaurate would less hydrophobic, and less miscible with the hydrophobic polyisobutylene diol.

In some embodiments, in addition to the sorbitan esters and/or the branched polypropylene oxide polyols, the crosslinking compounds may further include 1,1,1-*tris*(hydroxymethyl) propane or 1,2,3-trihydroxypropane. Such crosslinking compounds are not soluble with polyisobutylene diol by themselves because they lack any significant hydrophobic moieties. However, mixing the 1,1,1-*tris*(hydroxymethyl) propane or 1,2,3-trihydroxypropane with a sorbitan ester or a branched polypropylene oxide polyol may result in the combined crosslinking compounds being sufficiently hydrophobic to be miscible with the polyisobutylene diol. The sorbitan ester or the branched polypropylene oxide polyol acts as a "compatibilzing" agent, so that the 1,1,1-*tris*(hydroxymethyl) propane or 1,2,3-trihydroxypropane can participate in the polymerization process with the polyisobutylene diol. In some embodiments, the mixture of the crosslinking compounds is desiccated before being mixed with the polyisobutylene diol, the diisocyanate, and the polymerization catalyst, as described above.

A thermoset PIB-PUR in accordance with embodiments of this disclosure can be incorporated into medical devices which can be implanted or inserted into the body of a patient. Example medical devices may include, without limitation, electrical feedthroughs, optical feedthroughs, device casings, component casings (e.g., for ceramic capacitors, electrolytic capacitors, pressure sensors, transformers, inductors, etc.) for implantable electrical stimulation or diagnostic systems including cardiac systems such as implantable cardiac rhythm management (CRM) systems, implantable cardioverter-defibrillators (ICD's), cardiac resynchronization and defibrillation devices (CRDT), and leadless cardiac pacemakers (LCP), and for neurostimulation systems such as spinal cord stimulation (SCS) systems, deep brain stimulation (DBS) systems, peripheral nerve stimulation (PNS) systems, gastric nerve stimulation systems, cochlear implant systems, and retinal implant systems, among others.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

### EXAMPLES

The present invention is more particularly described in the following examples that are intended as illustrations only, since numerous modifications and variations within the scope of the present invention will be apparent to those of skill in the art. Unless otherwise noted, all parts, percentages, and ratios reported in the following examples are on a weight bases, and all reagents used in the examples were obtained, or are available, from the chemical suppliers described below, or may be synthesized by conventional techniques.

### Example 1

### Synthesis of Thermosetting Polyisobutylene-Polyurethane (PIB-PUR) with a Single Crosslinking Compound

PIB diol was combined with a 50/50 isomeric mixture of 4,4'-methylene diphenyl diisocyanate and 2,4'-methlene diphenyl diisocyanate (MDI) (Lupranate® MI available from BASF Corporation of Florham Park, NJ) in amounts indicated below in Table 1. About 0.004 g of tin(II) 2-ethylhexanoate was added to the PIB diol and diisocyanate, and the result combined in a high speed mixer at about 2000 rpm for 20 minutes. Pluracol® PEP450 (available from BASF Corporation of Florham Park, NJ) in amounts indicated below in Table 1 was combined with the mixture in a high-speed mixer at about 2000 rpm for 10 minutes.

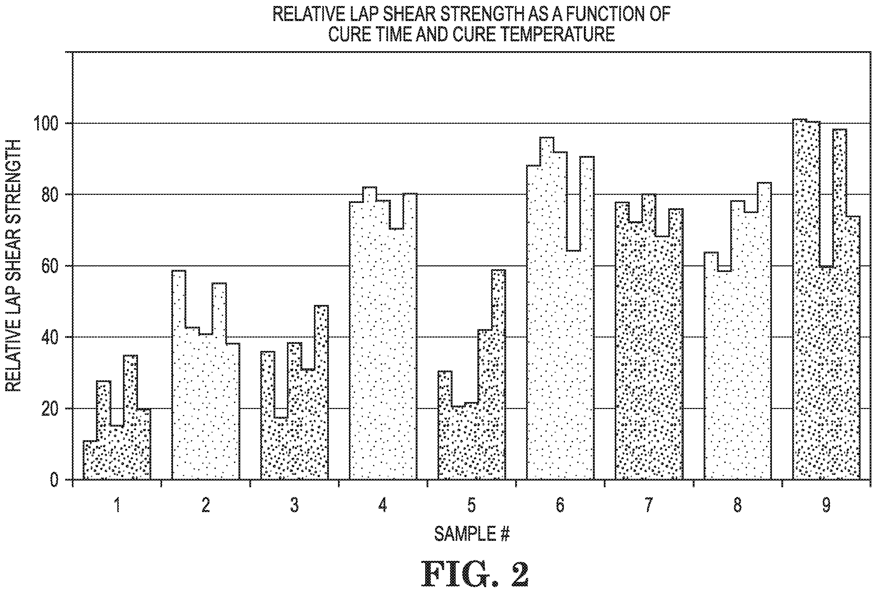

The resulting liquid mixture was applied to a portion of a surface of a titanium metal slide cleaned with heptane. Immediately, a second metal slide cleaned with heptane was placed on top of the liquid mixture in an overlapping arrangement with the first slide. The slide was cured for a time and at a temperature listed below in Table 1. The overlapping slides were subjected to lap shear strength test per ASTM D1002, except for that the test employed an overlap area of about 1 cm² and a bond thickness of about 0.25 mm to more closely correspond to anticipated use conditions. The results are shown in FIG. 2. As shown in FIG. 2, lap shear strength increases with both time and temperature, leveling off after about 8 hours at 100°C and after about 2 hours at 150°C. The highest lap shear strength was found at 16 hours at 100°C, suggesting that a longer cure time at a lower temperature provides the most robust mechanical strength.

**Table 1**

| Sample # | PIB diol (g) | MDI (g) | PEP450 (g) | Cure Time (hours) | Cure Temperature (°C) |
|---|---|---|---|---|---|
| 1 | 1.008 | 0.303 | 1.41 | 1 | 100 |
| 2 | 1.059 | 0.477 | 0.222 | 1 | 150 |
| 3 | 0.991 | 0.298 | 0.139 | 2 | 100 |
| 4 | 1.052 | 0.316 | 0.147 | 2 | 150 |
| 5 | 1.014 | 0.305 | 0.142 | 4 | 100 |
| 6 | 1.018 | 0.306 | 0.143 | 4 | 150 |
| 7 | 0.989 | 0.297 | 0.138 | 8 | 100 |
| 8 | 1.020 | 0.306 | 0.143 | 8 | 150 |
| 9 | 0.948 | 0.285 | 0.133 | 16 | 100 |

### Example 2

### Synthesis of Thermosetting Polyisobutylene-Polyurethane (PIB-PUR) with a Combination of Crosslinking Compounds

PIB diol was combined with a 50/50 isomeric mixture of 4,4'-methylene diphenyl diisocyanate and 2,4'-methlene diphenyl diisocyanate (MDI) (Lupranate® MI available from BASF Corporation of Florham Park, NJ) in amounts indicated below in Table 2. About 0.004 g of tin(II) 2-ethylhexanoate was added to the PIB diol and diisocyanate, and the result combined in a high speed mixer at about 2000 rpm for 20 minutes. Pluracol® PEP450 (available from BASF Corporation of Florham Park, NJ) and 1,1,1-*tris*(hydroxymethyl)propane (THP) (available from Sigma-Aldrich Co. LLC, St. Louis, MO) in amounts indicated below in Table 2 were combined in a high-speed mixer at about 2000 rpm for 10 minutes. The mixture of crosslinking compounds was then added to the PIB diol/diisocyanate mixture and combined in a high-speed mixer at about 2000 rpm for 10 minutes.

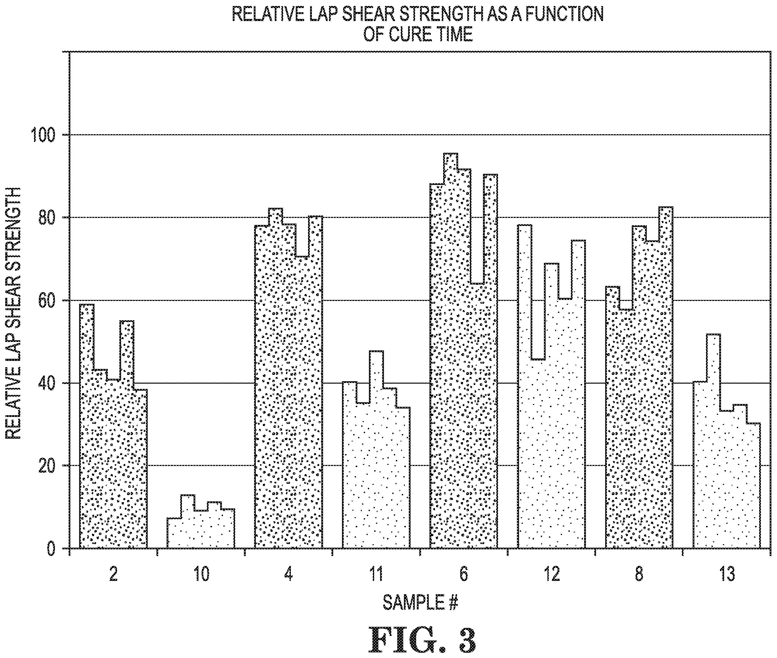

The resulting liquid mixture was applied to a portion of a surface of a titanium metal slide cleaned with heptane. Immediately, a second metal slide cleaned with heptane was placed on top of the liquid mixture in an overlapping arrangement with the first slide. The slide was cured at a temperature of 150°C and a time listed below in Table 2. The overlapping slides were subjected to lap shear strength test per ASTM D1002, except for that the test employed an overlap area of about 1 cm² and a bond thickness of about 0.25 mm to more closely correspond to anticipated use conditions. The results are shown in FIG. 3 along with the results using the single crosslinking compound cured at 150°C of Example 2. As shown in FIG. 3, a 50/50 molar ratio of PEP450 to THP reduces the lap shear strength compared to a PEP450 alone for each cure duration.

**Table 2**

| Sample # | PIB diol (g) | MDI (g) | PEP450 (g) | THP (g) | Cure Time (hours) |
|---|---|---|---|---|---|
| 10 | 1.042 | 0.313 | 0.073 | 0.024 | 1 |
| 11 | 1.005 | 0.311 | 0.072 | 0.024 | 2 |
| 12 | 1.005 | 0.302 | 0.070 | 0.024 | 4 |
| 13 | 1.011 | 0.304 | 0.071 | 0.024 | 8 |

## Claims

1. A thermoset polyisobutylene network polymer comprising:
a polyisobutylene diol residue;
a diisocyanate residue; and
at least one crosslinking compound residue selected from the group consisting of a residue of a sorbitan ester and a residue of a branched polypropylene oxide polyol.

2. The thermoset polymer of claim 1, wherein the polyisobutylene diol residue is present in the polymer in amount of 45% to 97.5% by weight of the polymer, the diisocyanate residue is present in the polymer in an amount of 2% to 30% by weight of the polymer, and the at least one crosslinking compound residue is present in the polymer in an amount of 0.5% to 25% by weight of the polymer.

3. The thermoset polymer of either of claims 1 or 2, wherein the diisocyanate residue includes 4,4'-methylene diphenyl diisocyanate residue and 2,4'-methylene diphenyl diisocyanate residue.

4. The thermoset polymer of claim 3, wherein the 2,4'-methylene diphenyl diisocyanate residue ranges from 1 wt. % to 50 wt. % of the diisocyanate residue.

5. The thermoset polymer of any of claims 1-4, wherein the crosslinking compound residue is a residue of a sorbitan ester with a hydroxyl functionality greater than 2.

6. The thermoset polymer of any of claims 1-4, wherein the crosslinking compound residue is a residue of a branched polypropylene oxide polyol with a hydroxyl functionality greater than 2.

7. The thermoset polymer of any of claims 1-5, and further including a residue of at least one of 1,1,1-*tris*(hydroxymethyl)propane and 1,2,3-trihydroxypropane.

8. The thermoset polymer of any of claims 1-6, wherein the polyisobutylene diol residue is a residue of α,ω-bishydroxy-terminated polyisobutylene.

9. A medical device comprising the thermoset polymer of any of claims 1-8.

10. The medical device of claim 9, wherein the thermoset polymer forms an electrically insulating and environmentally isolating portion of an electrical feedthrough.

11. A method of making a medical device including a thermoset polyisobutylene-polyurethane polymer, the method comprising:
mixing at a temperature ranging from 18°C to 30°C a polyisobutylene diol, a diisocyanate, a polymerization catalyst, and a crosslinking compound to form a liquid mixture, wherein the crosslinking compound including at least one member selected from the group consisting of a sorbitan ester having a hydroxyl functionality greater than 2 and a branched polypropylene oxide polyol having a hydroxyl functionality greater than 2;
applying the liquid mixture to the medical device; and
heating the medical device to cure the liquid mixture and form a thermoset polyisobutylene-polyurethane polymer on the medical device.

12. The method of claim 11, wherein applying the liquid mixture includes filling a space between a portion of the medical device and at least one electrical conductor with the liquid mixture, and the solid thermoset polyisobutylene-polyurethane polymer electrically insulates the electrical connection from the portion of the medical device.

13. The method of either of claims 11 or 12, wherein the diisocyanate includes 4,4'-methylene diphenyl diisocyanate ranging between 50 wt. % to 99 wt. % of the diisocyanate, and 2,4'-methylene diphenyl diisocyanate ranging between 1 wt. % and 50 wt. % of the diisocyanate.

14. The method of any of claims 11-13, further including desiccating the one or more crosslinking compounds before mixing together with the polyisobutylene diol, the diisocyanate, and the polymerization catalyst.

15. The method of claim 14, wherein the one or more crosslinking compounds further include of at least one of 1,1,1-*tris*(hydroxymethyl)propane and 1,2,3-trihydroxypropane; and further including mixing together the crosslinking compounds before desiccating the crosslinking compounds.

## Patentansprüche

1. Duroplastisches Polisobutylennetzwerk, umfassend:
einen Polyisobutylen-Diol-Rest;
einen Diisocyanat-Rest; und
mindestens einen Rest einer vernetzenden Verbindung, der ausgewählt ist aus der Gruppe bestehend aus einem Rest eines Sorbitanesters und einem Rest eines verzweigten Polypropylenoxid-Polyols.

2. Duroplastisches Polymer nach Anspruch 1, wobei der Polyisobutylen-Diol-Rest im Polymer in einer Menge von 45 bis 97,5 Gew.-% des Polymers vorliegt, der Diisocyanat-Rest im Polymer in einer Menge von 2 bis 30 Gew.-% des Polymers vorliegt und der mindestens eine Rest einer vernetzenden Verbindung im Polymer in einer Menge von 0,5 bis 25 Gew.-% des Polymers vorliegt.

3. Duroplastisches Polymer nach Anspruch 1 oder 2, wobei der Diisocyanat-Rest einen 4,4'-Methylen-Diphenyl-Diisocyanat-Rest und einen 2,4'-Methylen-Diphenyl-Diisocyanat-Rest umfasst.

4. Duroplastisches Polymer nach Anspruch 3, wobei sich der 2,4'-Methylen-Diphenyl-Diisocyanat-Rest von 1 Gew.-% bis 50 Gew.-% des Diisocyanat-Rest bewegt.

5. Duroplastisches Polymer nach einem der Ansprüche 1 bis 4, wobei der Rest einer vernetzenden Verbindung ein Rest eines Sorbitanesters mit einer Hydroxylfunktionalität größer als 2 ist.

6. Duroplastisches Polymer nach einem der Ansprüche 1 bis 4, wobei der Rest einer vernetzenden Verbindung ein Rest eines verzweigten Polypropylenoxid-Polyols mit einer Hydroxylfunktionalität größer als 2 ist.

7. Duroplastisches Polymer nach einem der Ansprüche 1 bis 5, ferner umfassend einen Rest von 1,1,1-Tris(hydroxymethyl)propan und/oder 1,2,3-Trihydroxypropan.

8. Duroplastisches Polymer nach einem der Ansprüche 1 bis 6, wobei der Polyisobutylen-Diol-Rest ein α,ω-Dihydroxy-terminierter Polyisobutylen-Rest ist.

9. Medizinische Vorrichtung, aufweisend das duroplastische Polymer nach einem der Ansprüche 1 bis 8.

10. Medizinische Vorrichtung nach Anspruch 9, wobei das duroplastische Polymer einen elektrisch isolierenden und einen gegenüber der Umwelt isolierenden Abschnitt einer elektrischen Durchführung bildet.

11. Verfahren zum Herstellen einer medizinischen Vorrichtung, die ein duroplastisches Polyisobutylen-Polyurethan-Polymer aufweist, wobei das Verfahren umfasst:
Vermischen, bei einer Temperatur zwischen 18 °C und 30 °C, eines Polyisobutylen-Diols, eines Diisocyanats, eines Polymerisationskatalysators und einer vernetzenden Verbindung, um ein Flüssigkeitsgemisch zu bilden, wobei die vernetzte Verbindung mindestens ein Element aus der Gruppe bestehend aus einem Sorbitanester mit einer Hydroxylfunktionalität größer als 2 und einem verzweigten Polypropylenoxid-Polyol mit einer Hydroxylfunktionalität größer als 2 umfasst;
Auftragen des Flüssigkeitsgemisches auf die medizinische Vorrichtung; und
Erwärmen der medizinischen Vorrichtung, um das Flüssigkeitsgemisch auszuhärten und auf der medizinischen Vorrichtung ein durplastisches Polyisobutylen-Polyurethan-Polymer zu bilden.

12. Verfahren nach Anspruch 11, wobei das Auftragen des Flüssigkeitsgemisches ein Befüllen eines Raumes zwischen einem Abschnitt der medizinischen Vorrichtung und mindestens einem elektrischen Leiter mit dem Flüssigkeitsgemisch umfasst, und das feste duroplastische Polyisobutylen-Polyurethan-Polymer die elektrische Verbindung vom Abschnitt der medizinischen Vorrichtung elektrisch isoliert.

13. Verfahren nach Anspruch 11 oder 12, wobei das Diisocyanat 4,4'-Methylen-Diphenyl-Diisocyanat in einer Menge zwischen 50 Gew.-% und 99 Gew.-% des Diisocyanats, und 2,4'-Methylen-Diphenyl-Diisocyanat in einer Menge zwischen 1 Gew.-% und 50 Gew.-% des Diisocyanats aufweist.

14. Verfahren nach einem der Ansprüche 11 bis 13, ferner umfassend ein Austrocknen der einen oder mehreren vernetzenden Verbindungen vor dem Vermischen mit dem Polyisobutylen-Diol, dem Diisocyanat und dem Polymerisationskatalysator.

15. Verfahren nach Anspruch 14, wobei die eine oder mehreren vernetzenden Verbindungen ferner 1,1,1-Tris(hydroxymethyl)propan und/oder 1,2,3-Trihydroxypropan aufweisen; und ferner umfassend ein Vermischen der vernetzenden Verbindungen vor dem Austrocknen der vernetzenden Verbindungen.

## Revendications

1. Polymère thermodurci réticulé à base de polyisobutylène comprenant :
un résidu de diol à base de polyisobutylène ;
un résidu de diisocyanate ; et
au moins un résidu de composé(s) de réticulation qui est sélectionné parmi le groupe qui est constitué par un résidu d'un ester de sorbitane et par un résidu d'un polyol à base d'oxyde de polypropylène ramifié.

2. Polymère thermodurci selon la revendication 1, dans lequel le résidu de diol à base de polyisobutylène est présent dans le polymère selon une quantité qui va de 45 % en poids à 97,5 % en poids du polymère, le résidu de diisocyanate est présent dans le polymère selon une quantité qui va de 2 % en poids à 30 % en poids du polymère et l'au moins un résidu de composé de réticulation est présent dans le polymère selon une quantité qui va de 0,5 % en poids à 25 % en poids du polymère.

3. Polymère thermodurci selon l'une ou l'autre des revendications 1 et 2, dans lequel le résidu de diisocyanate inclut un résidu de diisocyanate de 4,4'-méthylène diphényle et un résidu de diisocyanate de 2,4'-méthylène diphényle.

4. Polymère thermodurci selon la revendication 3, dans lequel le résidu de diisocyanate de 2,4'-méthylène diphényle s'inscrit dans la plage qui va de 1 % en poids à 50 % en poids du résidu de diisocyanate.

5. Polymère thermodurci selon l'une quelconque des revendications 1 à 4, dans lequel le résidu de composé(s) de réticulation est un résidu d'un ester de sorbitane qui présente une fonctionnalité hydroxyle supérieure à 2.

6. Polymère thermodurci selon l'une quelconque des revendications 1 à 4, dans lequel le résidu de composé(s) de réticulation est un résidu d'un polyol à base d'oxyde de polypropylène ramifié qui présente une fonctionnalité hydroxyle supérieure à 2.

7. Polymère thermodurci selon l'une quelconque des revendications 1 à 5, et incluant en outre un résidu d'au moins un composé pris parmi le 1,1,1-*tris*(hydroxyméthyl)propane et le 1,2,3-trihydroxypropane.

8. Polymère thermodurci selon l'une quelconque des revendications 1 à 6, dans lequel le résidu de diol à base de polyisobutylène est un résidu de polyisobutylène terminé par α,ω-bishydroxy.

9. Dispositif médical comprenant le polymère thermodurci selon l'une quelconque des revendications 1 à 8.

10. Dispositif médical selon la revendication 9, dans lequel le polymère thermodurci forme une partie électriquement isolante et environnementalement isolante d'une alimentation par interconnexion électrique.

11. Procédé de fabrication d'un dispositif médical qui inclut un polymère thermodurci à base de polyisobutylène-polyuréthane, le procédé comprenant :
le mélange, à une température qui s'inscrit dans la plage qui va de 18 °C à 30 °C, d'un diol à base de polyisobutylène, d'un diisocyanate, d'un catalyseur de polymérisation et d'un composé de réticulation pour former un mélange liquide, dans lequel le composé de réticulation inclut au moins un élément qui est sélectionné parmi le groupe qui est constitué par un ester de sorbitane qui présente une fonctionnalité hydroxyle supérieure à 2 et par un polyol à base d'oxyde de polypropylène ramifié qui présente une fonctionnalité hydroxyle supérieure à 2 ;
l'application du mélange liquide sur le dispositif médical ; et
le chauffage du dispositif médical pour durcir le mélange liquide et pour former un polymère thermodurci à base de polyisobutylène-polyuréthane sur le dispositif médical.

12. Procédé selon la revendication 11, dans lequel l'application du mélange liquide inclut le remplissage d'un espace entre une partie du dispositif médical et au moins un conducteur électrique à l'aide du mélange liquide, et le polymère thermodurci à base de polyisobutylène-polyuréthane solide isole électriquement la connexion électrique vis-à-vis de la partie du dispositif médical.

13. Procédé selon l'une ou l'autre des revendications 11 et 12, dans lequel le diisocyanate inclut du diisocyanate de 4,4'-méthylène diphényle selon une proportion s'inscrivant dans la plage entre 50 % en poids et 99 % en poids du diisocyanate et du diisocyanate de 2,4'-méthylène diphényle selon une proportion s'inscrivant dans la plage entre 1 % en poids et 50 % en poids du diisocyanate.

14. Procédé selon l'une quelconque des revendications 11 à 13, incluant en outre la dessiccation des un ou plusieurs composés de réticulation avant leur mélange ensemble avec le diol à base de polyisobutylène, le diisocyanate et le catalyseur de polymérisation.

15. Procédé selon la revendication 14, dans lequel les un ou plusieurs composés de réticulation incluent en outre au moins un composé pris parmi le 1,1,1-*tris*(hydroxyméthyl)propane et le 1,2,3-trihydroxypropane ; et incluant en outre le mélange ensemble des composés de réticulation avant la dessiccation des composés de réticulation.
